# EUROPÄISCHE PATENTSCHRIFT

(11) **EP 4 323 018 B1**
(45) Veröffentlichungstag und Bekanntmachung des Hinweises auf die Patenterteilung: **23.04.2025**
(21) Anmeldenummer: 22716372.2
(22) Anmeldetag: 17.03.2022
(51) Int. Cl.: A61K 51/08, A61K 51/12

(54) **HERSTELLUNG VON RADIOPHARMAKA FÜR UNTERSCHIEDLICHE ANWENDUNGSZEITPUNKTE**
PREPARATION OF RADIOPHARMACEUTICALS FOR DIFFERENT TIMES OF APPLICATION
PRÉPARATION DE PRODUITS RADIOPHARMACEUTIQUES POUR DIFFÉRENTS TEMPS D'APPLICATION

(30) Priorität: 13.04.2021 DE 102021109246
(43) Veröffentlichungstag der Anmeldung: 21.02.2024
(73) Patentinhaber: ITM Isotope Technologies Munich SE, 85748 Garching bei München (DE)
(72) Erfinder: MECKEL, Marian, 85354 Freising (DE); KOZIOROWSKI, Jacek, 85354 Freising (DE); HARFENSTELLER, Mark, 85716 Unterschleißheim (DE); SCHNEIDER, Karl-Heinz, 85667 Oberpframmern (DE)
(74) Vertreter: Winter, Brandl - Partnerschaft mbB
(86) Internationale Anmeldenummer: PCT/EP2022/057040
(87) Internationale Veröffentlichungsnummer: WO 2022/218640

(56) Entgegenhaltungen:
- DE-A1- 10 040 771
- RITAWIDYA RIEN ET AL: "Synthesis of DOTA-TOC Conjugate as a Precursor of 177Lu-DOTA-TOC Radiopharmaceutical for Therapy and Diagnosis of Somatostation Receptor Positive Cancer", INDONESIAN POLYMER JOURNAL, vol. 19, no. 1, 1 July 2016 (2016-07-01), pages 1 - 14, XP055942185, Retrieved from the Internet <URL:http://hpi-polimer.org/images/MPI/19-1-2016/1-14_RRitawidya.pdf>

## Beschreibung

Die vorliegende Erfindung betrifft ein Verfahren zur Herstellung von Radionukliden enthaltenden Produkten mit einer gleichen gewünschten Aktivität der Radioaktivität zu unterschiedlichen Anwendungszeitpunkten, bezogen auf einen gegebenen Kalibrierzeitpunkt gemäß dem Oberbegriff des Anspruchs 1 sowie eine Vorrichtung zur Durchführung des Verfahrens gemäß Anspruch 19. Radiopharmazeutika sind radioaktive Verbindungen, die in der Nuklearmedizin angewendet werden. Man unterscheidet diagnostische und therapeutische Radiopharmaka oder sogenannte Theranostika, welche sowohl therapeutisch als auch diagnostisch eingesetzt werden können. Solche Arzneimittel werden sehr häufig direkt vor Ort ihrer Nutzung hergestellt, was auf Grund ihrer kurzen Halbwertszeit bzw. Lebensdauer, insbesondere bei diagnostischen Verbindungen, notwendig erscheint. Komplexe therapeutische Radiopharmaka wurden bis vor wenigen Jahren häufig ebenfalls nur in geringen Mengen patientenorientiert lokal hergestellt. Lediglich einfach darstellbare, weniger komplexe Radiopharmaka, die entweder nur aus einem Radionuklid oder einer einfachen Radionuklidformulierung bestehen wurden bis dato zentralisiert hergestellt und zu den nuklearmedizinischen Anwendern verteilt. Zu solchen Arzneimitteln zählen beispielsweise [I-131]NaI, [Ra-223]RaCl₂ und [Sm-153]Sm-EDTMP.

Mittlerweile stehen dem Fachmann eine Reihe von geeigneten radioaktiven Isotopen in ausreichender Menge und pharmazeutischer Qualität zur Verfügung. Nur beispielhaft sei hier auf die in jüngerer Zeit bedeutsamen Isotope Lu-177 und Ga-68 verwiesen:
So beschreibt beispielsweise die Anmelderin der vorliegenden Patentanmeldung in EP2546839B1 ein bereits patentiertes Verfahren zur Herstellung trägerfreier hochreiner Lu-177 Verbindungen (Halbwertszeit = 6.64 Tage, ß⁻ - Zerfall) für medizinische Zwecke.

Dort wird insbesondere ein präparatives säulenchromatographisches Verfahren zur Herstellung derartiger trägerfreier, hochreiner ¹⁷⁷Lu-Verbindungen offenbart. Bei dem ¹⁷⁷Lu-Herstellungsverfahren kommen ein Kationenaustauscher und ein geeigneter Komplexbildner zum Einsatz. Mit dem genannten Verfahren ist es erstmals möglich, aus mit thermischen Neutronen bestrahlten ¹⁷⁶Yb-Verbindungen, wobei die Radionuklide ¹⁷⁷Lu und ¹⁷⁶Yb für die Aufreinigung in einem Massenverhältnis von ca. 1:10² bis 1:10¹⁰ vorliegen, trägerfreie, hochreine ¹⁷⁷Lu-Verbindungen in Milligrammmengen für pharmazeutisch-medizinische Zwecke in hochreiner Form zur Verfügung zu stellen.

Darüber hinaus offenbart die WO/2018/122250A1 der Anmelderin der vorliegenden Patentanmeldung einen ⁶⁸Ge/⁶⁸Ga - Generator mit welchem der Positronenstrahler Ga-68 (Halbwertszeit = 67.71 Minuten) kontinuierlich vor Ort, z.B. im nuklearmedizinischen Labor einer Klinik in pharmazeutischer Qualität für die Herstellung von Theranostika erzeugt werden kann.

Eine allgemeine Übersicht über stabile konzentrierte Radionuklidkomplex-Lösungen des Standes der Technik gibt beispielsweise US 10596278B2.

Chelatorkomponenten für Radionuklide und Targetkomponenten sind beispielsweise in EP 1 289 571 B1 ausführlich beschrieben und dem Fachmann somit wohlbekannt. Die genannte Druckschrift betrifft Prochelatoren und Chelatoren von Radiometall-markierten Molekülen im Allgemeinen.

Dort sind makrocyclische Polyazaverbindungen zur Markierung mit radioaktiven Metallen beschrieben, enthaltend ein Nₙ-System, wobei n für 4, 5 oder 6 steht, mit verschiedenen Ringgrößen, und wobei wenigstens eines der N-Atome für die Kupplung an eine Aminofunktion in einem biologisch aktiven Effektormolekül durch eine freie Carboxylgruppe substituiert ist, wobei zur Synthese des endfertigen Moleküls sämtliche N-Atome eine geschützte Seitenkette tragen.

Insbesondere beschreibt EP 1 289 571 B1 einen Chelatbildner zur Markierung von biologisch aktiven Molekülen mit einem radioaktiven Metall, mit der allgemeinen Formel: 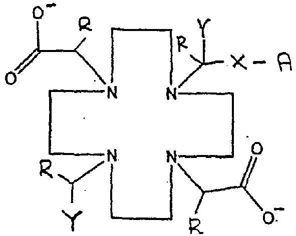 wobei:
die beiden Y-Gruppen entweder, wie gezeigt, trans oder cis angeordnet sein können;
A für ein Effektormolekül wie ein Peptid, insbesondere Octreotid, CCK, Substanz P oder Gastrin, ein Protein, insbesondere einen Antikörper oder ein Enzym,
einen Zucker oder ein radiosensibilisierendes Mittel wie Doxorubicin steht;
R für Wasserstoff, C₁-C₃-Alkyl oder einen Alkohol steht;
X für einen Spacer, insbesondere (CH₂)ₙ-X', steht, wobei n für 1-10 steht und X' für COOH, NH₂, SH, OH oder O-Halogen steht, wobei es sich bei dem Halogen insbesondere um Br, I oder Cl handelt,
oder ein Molekül der Formel 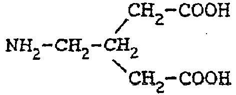
oder der Formel 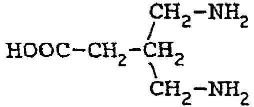 steht,
Y für COO⁻, CH₂CONH₂ oder CH₂CH₂OH steht, gegebenenfalls komplexiert mit einem radioaktiven Metall.

Darüber hinaus gibt BREEMAN (2012) [Wouter A. P. BREEMAN; Practical Aspects of labeling DTPA- and DOTA-Peptides with 90Y, 111In, 177Lu, and 68Ga for Peptide-Receptor Scintigraphy and Peptide-Receptor Radionuclide Therapy in Preclinical and Clinical Applications The University of New Mexico Health Sciences Center, VOLUME 16, LESSON 5: 11/16/2012] einen Überblick über praktische Aspekte der Markierung von DTPA- und DOTA-Peptiden mit ⁹⁰Y, ¹¹¹In, ¹⁷⁷Lu, und ⁶⁸Ga für die Peptid-Rezeptor-Scintigraphie und Peptide-Receptor Radionuklidtherapie in präklinischen und klinischen Anwendungen.

HEPPELER et al. (1999) beschreiben Somatostatinanaloga, die mit radiometall-markierten makrozyklischen Chelatorkomponenten derivatisiert sind [HEPPELER et al.: "Radiometal-labelled macrocyclic chelator-derivatized somatostatin analogue with superb tumour-targeting properties and potential for receptor-mediated internal radiotherapy", Chem.-Eur. J., 1999, 5(7), 1974-1981].

EISENWIENER et al. (2001) offenbaren eine Synthese und Peptidkupplung eines auf DOTA basierenden Prochelators, der neutrale Komplexe mit Yttrium-90 and Indium-111 bildet. [Eisenwiener et al.: "Synthesis and peptide coupling of a new DOTA based prochelator, forming neutral complexes with Yttrium-90 and Indium-111", Journal of Labelled Compounds and Radiopharmaceuticals, May 2001, Vol. 44, No. Supplement 1, pp. S694-S696. PRINT. Meeting Info: 14th International symposium on Radiopharmaceutical chemistry Interlaken, Switzerland, June 10-15, 2001].

ANDRÉ et al. (1998) beschreiben 1,4,7-Triazacyclononan-1-bernsteinsäure-4,7-diessigsäure (NODASA) als bifunktionellen Chelator für mit radioaktivem Gallium markierte Biomoleküle [André, J. et al.: "1,4,7-Triazacyclononane-1-succinic acid-4,7-diacetic acid (NODASA): a new bifunctional chelator for radio gallium-labelling of biomolecules", Chem. Commun., 1998, 12, 1301-1302].

RIEN et al. (2016) beschreiben die Radionuklidmarkierung von DOTATOC mit Lu in einer Pufferlösung [Rien et al.: "Synthesis of DOTA-TOC Conjugate as a Precursor of 177Lu-DOTA-TOC Radiopharmaceutical for Therapy and Diagnosis of Somatostatin Receptor Positive Cancer", Indonesian Polymer Journal, 2016, 19(1), 1-14].

Somit steht dem Fachmann eine Vielzahl von unterschiedlichen Radionukliden und hierfür geeignete Chelatorkomponenten und Targetmolekülkomponenten sowie Markierungstechniken und die Verwendung der markierten Moleküle für medizinische Zwecke zur Verfügung.

Für therapeutische Radiopharmaka, welche - wie oben beschrieben - aus äußerst komplexen Formulierungen und Komponenten bestehen, existieren verschiedene Herausforderungen in ihrer zentralisierten Herstellung. Durch den Zerfall der in der Regel kurzlebigen radioaktiven Komponente ergibt sich beispielsweise die Schwierigkeit, die gleiche Zusammensetzung des Arzneimittels zu allen Anwendungszeitpunkten in Bezug auf einen Kalibrierzeitpunkt (Activity Reference Time, ART) zu gewährleisten. Dieses Problem wird aktuell und wurde in der Vergangenheit durch sogenannte Kit-Rekonstitutionen zum Anwendungszeitpunkt gelöst. Hierbei wird die radioaktive Komponente des Arzneimittels separat zur Verfügung gestellt und es muss vor Anwendung eine Rekonstitution oder gar eine komplexe Synthese sowie eine Qualitätskontrolle vor Ort durchgeführt werden. Beispiele hierfür sind Octreoscan ([In-111]In-Pentetreotid) und Zevalin ([Y-90]Y-Ibritumomab-Tiuxetan).

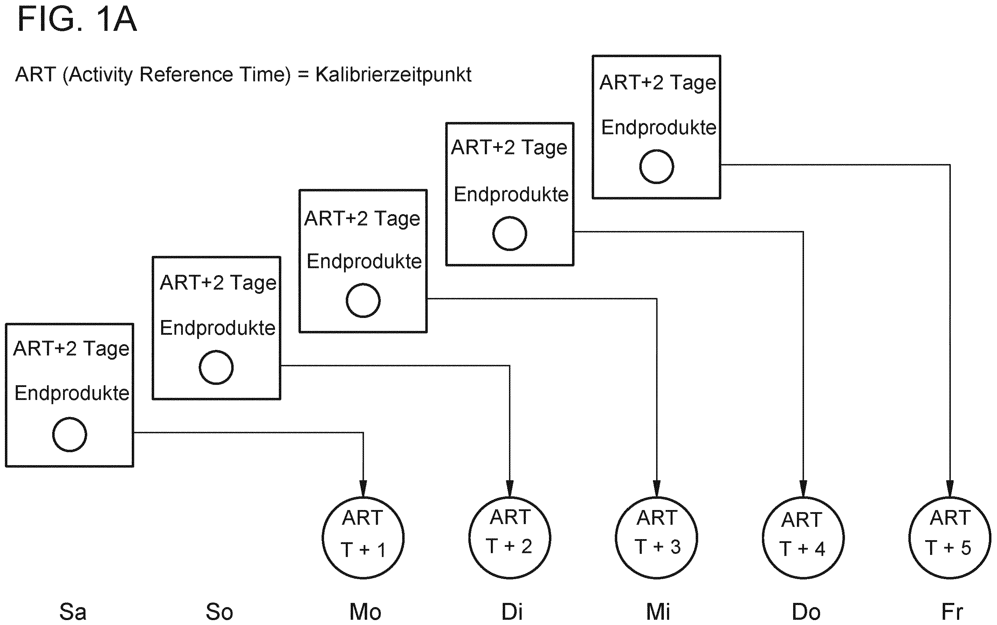

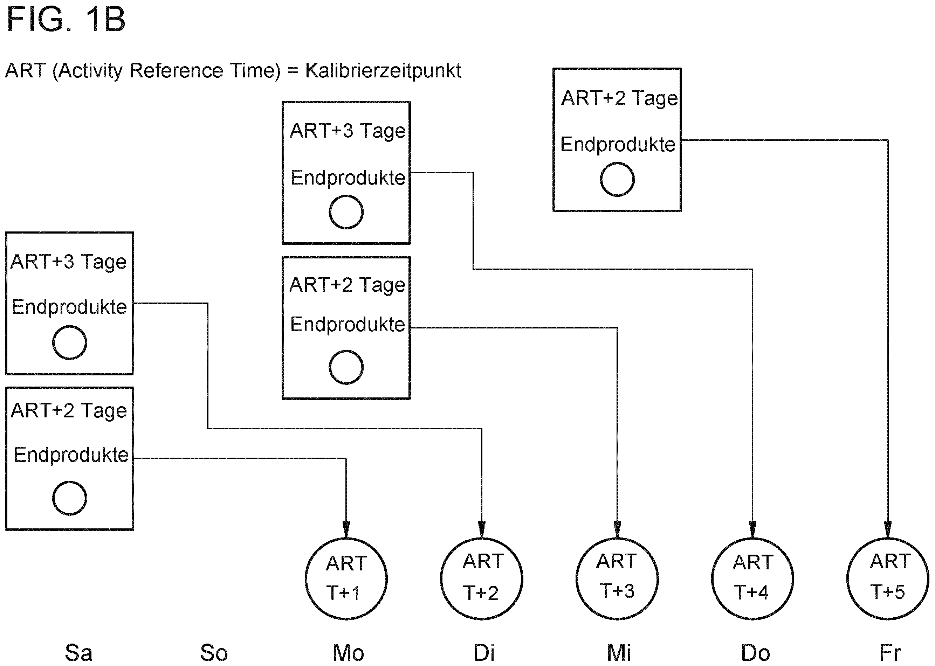

Eine andere Möglichkeit, diese Schwierigkeit zu umgehen besteht darin, das Radiopharmazeutikum für einen festen Anwendungszeitpunkt herzustellen und dem Anwender bereitzustellen. Je nach Konzeption ergeben sich dadurch Nachteile entweder für den Hersteller oder den Anwender. Um das Arzneimittel jeden Tag in der Arbeitswoche (Montag bis Freitag) zur Verfügung stellen zu können, müsste der Hersteller mindestens 5 separate Kleinchargen pro Woche zu einem fixen Anwendungszeitpunkt avisieren mit einer entsprechenden Produktionsplanung und Organisationsvorlauf für den Bestellprozess. Eine solche Produktionsweise des Standes der Technik ist in Fig. 1 gezeigt. Dieses Verfahren ist insbesondere aus regulatorischen Gründen für eine Arzneimittelzulassung für den Hersteller aufwändig und kostenintensiv, da jede einzelne Arzneimittel-Tagescharge - neben der Prüfung der korrekt eingesetzten Radioaktivitätsmenge - mit intensiver Qualitätskontrolle und arzneimittelgesetzlicher Freigabe verbunden ist. Eine bestehende Produktionsanlage wäre bei Realisierung eines solchen Konzeptes vollständig ausgelastet und könnte nur schwer für die Herstellung anderer Radiopharmazeutika eingesetzt werden.

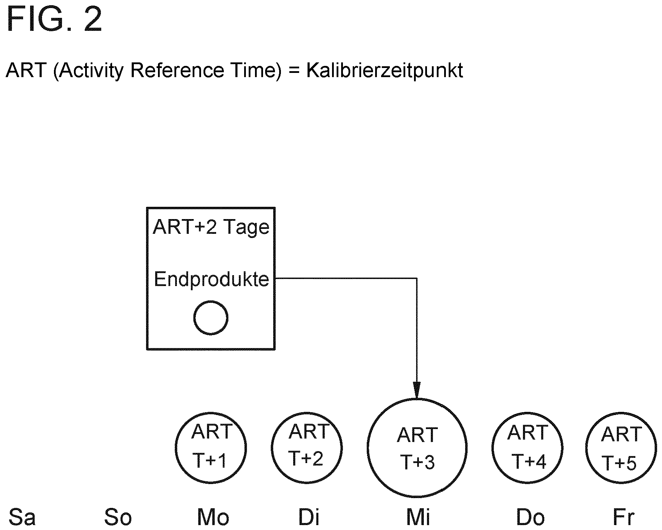

Ein alternatives Konzept des Standes der Technik ist die Herstellung einer einzigen großen Charge pro Woche vorsehen. Dadurch spart sich der Hersteller die oben genannten Nachteile und würde zudem kostengünstiger produzieren. Nachteilig an diesem Procedere ist jedoch, dass der Anwender das Radiopharmakon nur zu vordefinierten, vom Hersteller bestimmten Zeiten, zur Verfügung gestellt bekommt und der Anwender muss seine gesamte Planung dem Hersteller unterordnen. Diese Situation ist besonders für den klinischen Anwender von Nachteil, was in Folge zu einer geringeren Akzeptanz des entsprechenden radiopharmazeutischen Produktes führen kann und erfahrungsgemäß auch führt. Ein solches Produktionsregime ist schematisch in Fig. 2 gezeigt.

Eine weitere Alternative besteht in der individualisierten Herstellung der Radiopharmaka zu einem definierten Gebrauchszeitpunkt. Über Zerfallsberechnungen des jeweilig eingesetzten Isotops kann zu einem definierten Zeitpunkt vor dem Gebrauchszeitpunkt so viel weniger Lösung entnommen werden, dass die Aktivität der Aktivität zum Sollzeitpunkt entspricht. Allerdings sind bei dieser Vorgehensweise weder die Radioaktivitätskonzentration noch die Konzentration der darin enthaltenen chemischen Verbindungen damit nicht konstant und auch die Menge des Target-Biomoleküls ist veränderlich. Dies kann zu einer suboptimalen Aufnahme des Medikaments im Patienten führen.

Vor diesem Hintergrund ist es Aufgabe der vorliegenden Erfindung, zu einem bestimmten Anwendungszeitpunkt eine exakte und gleiche Radioaktivitätsmenge in radiopharmazeutischen Produkten, bezogen auf einen Kalibrierzeitpunkt in einer Mehrzahl von einzelnen Chargen während der Arbeitswoche zur Verfügung zu stellen, ohne die oben geschilderten Nachteile in Kauf nehmen zu müssen.

Die Lösung dieser Aufgabe erfolgt durch ein Verfahren mit den kennzeichnenden Merkmalen des Anspruchs 1.

Vorrichtungstechnisch wird die Aufgabe durch eine Vorrichtung gemäß Anspruch 19 gelöst.

Insbesondere betrifft die vorliegende Erfindung ein Verfahren zur Herstellung von Radionuklide enthaltenden Produkten mit einer gleichen gewünschten Aktivität der Radioaktivität zu unterschiedlichen Anwendungszeitpunkten (ART+1, ART+2, ART+3, ART+4), bezogen auf einen gegebenen Kalibrierzeitpunkt (ART), wobei
- ein Radionuklid enthaltendes Konzentrat zu einem gewünschten mit dem Radionuklid markierten Produkt umgesetzt wird und so eine Stammlösung erhalten wird, welche neben dem radionuklidmarkierten Produkt sämtliche für den Anwendungszweck benötigten weiteren Bestandteile enthält, wobei
- das gewünschte Radionuklid in der Stammlösung in einer solchen Aktivität enthalten ist, dass eine Mehrzahl von gewünschten Chargen mit jeweils einer definierten Anzahl von Teilabfüllungen zu einem Abfüllzeitpunkt aus der Stammlösung erhältlich ist, wobei jede Charge von Teilabfüllungen zu unterschiedlichen Anwendungszeitpunkten (ART+1, ART+2, ART+3, ART+4) jeweils eine gleiche Aktivität des Radionuklids, bezogen auf den Kalibrierzeitpunkt (ART), aufweist;
- die Aktivität des radionuklidmarkierten Produktes in der Stammlösung auf einen spätesten gewünschten Anwendungszeitpunkt (ART+4) eingestellt wird;
- aus der das radionuklidmarkierte Produkt enthaltenden Stammlösung eine erste Charge von Teilabfüllungen zu einem ersten vor dem Anwendungszeitpunkt liegenden Abfüllzeitpunkt entnommen wird, welche

eine auf den spätesten Anwendungszeitpunkt (ART+4) eingestellte Aktivität aufweist, welche zu ihrem tatsächlichen Anwendungszeitpunkt der Aktivität zum Kalibrierzeitpunkt (ART) entspricht;
- eine Verdünnungslösung bereitgestellt wird, welche mit Ausnahme des radionuklidmarkierten Produktes sämtliche für den Anwendungszweck benötigten weiteren Bestandteile enthält;
- die verbleibende, auf den spätesten gewünschten Anwendungszeitpunkt (ART+4) eingestellte Stammlösung mit der Verdünnungslösung derart verdünnt wird, dass zum Abfüllzeitpunkt eine gewünschte verminderte Aktivität, bezogen auf den spätesten Anwendungszeitpunkt (ART+4) eingestellt wird, so dass für den Einsatz zum vorhergehenden Anwendungszeitpunkt eine zweite Charge von Teilabfüllungen entnommen wird, welche eine auf einen früheren Anwendungszeitpunkt (ART+3) eingestellte Aktivität aufweist, welche zu ihrem tatsächlichen Anwendungszeitpunkt der Aktivität zum Kalibrierzeitpunkt (ART) entspricht;
- die verbleibende, auf den früheren Anwendungszeitpunkt (ART+3) eingestellte Stammlösung mit der Verdünnungslösung schrittweise solange weiter verdünnt wird, bis der Anwendungszeitpunkt dem Kalibrierzeitpunkt (ART) entspricht; und
- jeweils weitere Chargen von Teilabfüllungen zu jedem weiteren Anwendungszeitpunkt (ART+2, ART+1) entnommen werden, welche eine auf den jeweiligen Anwendungszeitpunkt (ART+2, ART+1) eingestellte Aktivität aufweisen, wobei die letzte Charge die Aktivität des Kalibrierzeitpunktes (ART) aufweist.

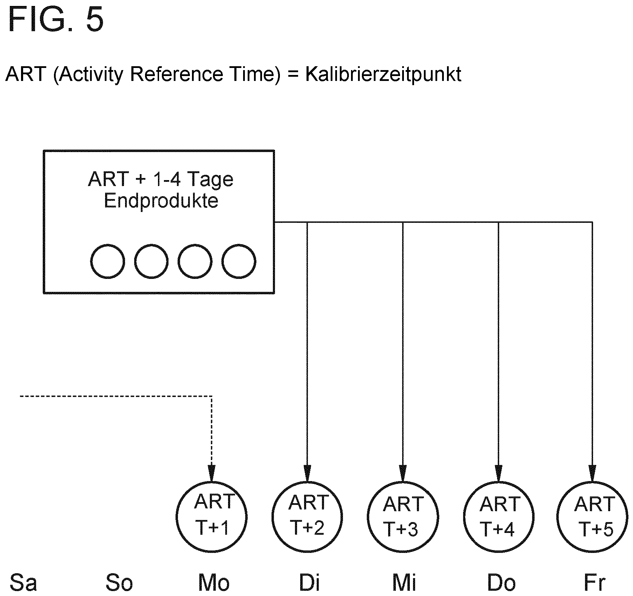

Die Erfindung ermöglicht die Herstellung des pharmazeutischen Präparates Solucin^{®} (eingetragene Marke der ITM Isotopen Technologien München AG) in einem einzigen Herstellansatz zu allen möglichen ARTs in einer kleinen und kompakten Anlage, welche die Abfüllung mit einschließt. Dadurch werden im Vergleich zum aktuellen Stand der Technik deutlich weniger Ansätze benötigt (vgl. Fig. 5). Der Hersteller spart sich dadurch Herstell-, Prüf- und Freigabekosten, ohne die Verfügbarkeit des Arzneimittels einzuschränken. Außerdem gewinnt man zusätzliche Kapazitäten an der Produktionsanlage für andere Produkte, was eine Produktivitäts- und Effizienzsteigerung bedeutet. Für die Bulkansätze besteht kein theoretisches Limit. Im Gegensatz zur Alternative 1 kann in der erfindungsgemäßen Alternative 2 auf eine zweite Abfülleinheit verzichtet werden, was wiederum Investitions-, Wartungs- und Qualifizierungskosten spart.

Die vorliegende Erfindung betrifft ferner eine Vorrichtung zur Durchführung des erfindungsgemäßen Verfahrens mit einem Fluidiksystem, an welches die folgenden Bauteile angeschlossen sind:
wenigstens ein Reaktor;
ein einstellbares Heizelement, das zum Heizen des Reaktors dient;
eine einstellbare Vakuumpumpe mit Pneumatik und Entlüftungsventilen;
eine einstellbare Inertgaspneumatik;
ein Behälter für eine Formulierungslösung, welcher fluidisch mit dem Reaktor verbunden ist;
ein Behälter für eine Verdünnungslösung;
einem Reaktionspufferbehälter;
ein Vorlagebehälter für einen radiochemischen Präkursor;
eine Abfülldosiereinrichtung;
ein Bulkaufbewahrungs- und Mischgefäß;
ein belüfteter Sterilfilter;
Luftfilter;
eine By-Pass-Leitung zwischen unsteriler Seite des Sterilfilters und dem über einen Dreiwegehahn damit fluidisch verbundenen Bulkaufbewahrungs- und Mischgefäß;
eine Abfülleinrichtung; sowie
eine erste Hahnenbank mit Mehrwegeventilen; und
eine zweite Hahnenbank mit Mehrwegeventilen;
wobei die erste Hahnenbank in fluidischer Verbindung mit dem Luftfilter, dem Bulkaufbewahrungs- und Mischgefäß, der
Inertgaspneumatik, dem Behälter, dem Sterilfilter sowie der Abfülldosiereinrichtung steht; und
wobei die zweite Hahnenbank in fluidischer Verbindung mit dem Reaktor, dem Vorlagenbehälter, dem Reaktionspufferbehälter, der By-Pass-Leitung, dem Bulkaufbewahrungs- und Mischgefäß und dem Luftfilter steht wobei das Bulkaufbewahrungs- und Mischgefäß in fluidischer Verbindung mit der Vakuumpumpe steht.

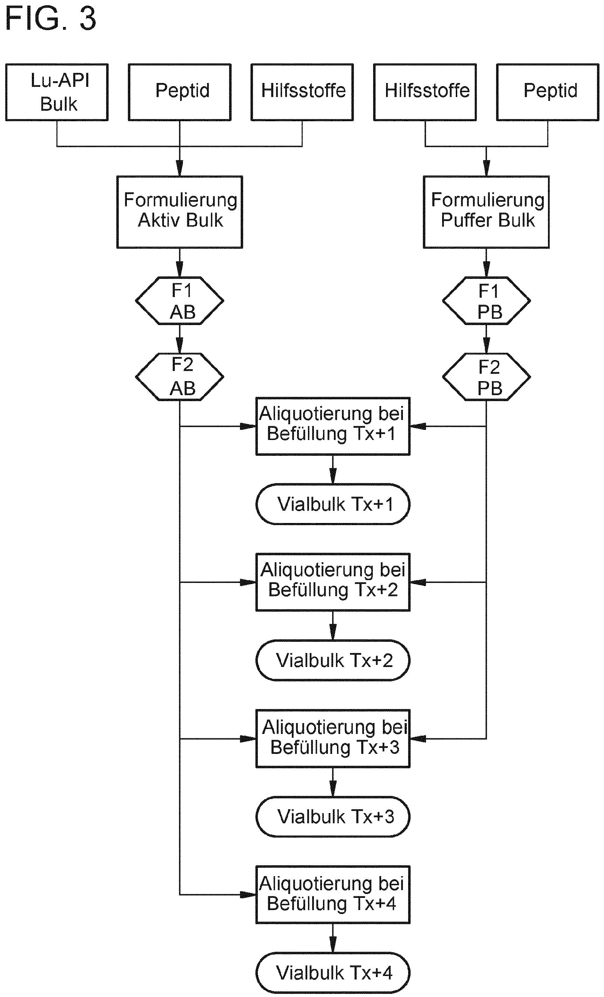

Grundsätzlich wäre es zwar möglich Radiopharmazeutika für unterschiedliche Anwendungszeitpunkte gemäß einer Alternative 1 folgendermaßen herzustellen:
Es wird ein Konzentrat zum spätesten Kalibrierzeitpunkt (ART+4) hergestellt. Aus diesem Konzentrat wird der ART+4 direkt zu 100% abgefüllt. Zusätzlich kann aus diesem Konzentrat mit einer Verdünnungslösung alle anderen ARTs (ART1-3) dargestellt werden, in dem entsprechend weniger des Konzentrats in die jeweiligen Vials abgefüllt wird und mit einer Verdünnungslösung auf das spezifikationskonforme Volumen aufgefüllt wird. Hierfür wären jedoch zwei entsprechende Abfülleinheiten in der Abfülllinie notwendig, nämlich jeweils eine Abfülllinie für das Konzentrat und eine für die Verdünnungslösung. Nachteilig wäre dabei jedoch, dass jedes Vial unter arzneimittelrechtlichen Aspekten bzw. nach GMP ein Unikat darstellen würde und insbesondere für die pharmazeutische Praxis erforderlichen Qualitätskontrollen könnte sich dadurch ein nicht repräsentativer Probenzug ergeben. Die Homogenisierung muss außerdem im jeweiligen Vial erfolgen. Dies würde einen großen Validierungsaufwand für den Abfüllprozess bedeuten und wäre unter den erwähnten arzneimittelrechtlichen Aspekten aufwendig und eher unpraktikabel. Das Abfüllschema gemäß Alternative 1 des Standes der Technik ist in Fig. 3 gezeigt.

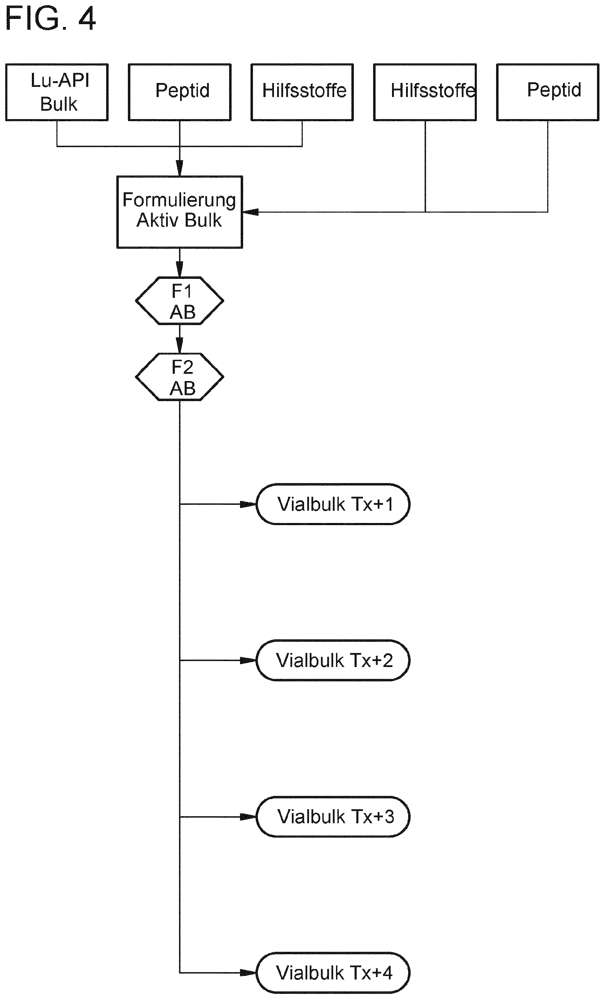

Hier setzt die Erfindung ein (Alternative 2):
Erfindungsgemäß wird ein Konzentrat zum spätesten Kalibrierzeitpunkt (ART+4) hergestellt. Aus diesem Konzentrat wird der ART+4 direkt und zu 100% abgefüllt. Zusätzlich kann aus diesem Konzentrat mit einer Verdünnungslösung alle anderen ARTs (ART1-3) dargestellt werden. Hierfür werden aus dem Bulkgefäß zuerst alle ART+4 abgefüllt. Anschließend erfolgt - im Gegensatz zu den oben erwähnten Einzelverdünnungsabfüllungen in Vials - eine Verdünnung des Bulkansatzes zum Art+3 mit anschließender Abfüllung ebendieser. Schließlich folgt ART+2 und ART+1 etc. Der Vorteil bei dem erfindungsgemäßen Konzept liegt in der deutlich kleineren Anlage die lediglich aus einer einzigen Abfülleinheit in der Abfülllinie besteht. Außerdem erfolgen die einzelnen Abfüllungen aus einem homogenen Bulkansatz. Dadurch ergibt sich für den Probenzug für die Qualitätskontrolle ein deutlich homogeneres Bild. Ein Abfüllschema des Verfahrens der vorliegenden Erfindung gemäß Alternative 2 ist in Fig. 4 gezeigt.

Im Folgenden sind bevorzugte Ausführungsformen der vorliegenden Erfindung beschrieben:
Besonders vorteilhaft kann das erfindungsgemäße Verfahren mit sämtlichen derzeit relevanten Radionukliden durchgeführt werden. Dies sind beispielsweise solche, welche ausgewählt werden, aus der Gruppe, bestehend aus: Gallium-68, Yttrium-90, Molybdän-99, Indium-111, Gadolinium-146, Gadolinium 147, Holmium-166, Lutetium-177, Wolfram-188, Rhenium-188, Bismut-205, Bismut-206 und Thorium-227.

Auf Basis der genannten kurzlebigen Radionuklide hergestellte Theranostika - also therapeutisch und/oder diagnostisch einsetzbare Substanzen - haben sich bereits vielfach in der Nuklearmedizin bewährt und können mit dem erfindungsgemäßen Verfahren mit jeweils exakt kalibrierter Aktivität, in ausreichender Menge und gleichbleibend hoher Qualität für sämtliche Anwendungszeitpunkte innerhalb einer Arbeitswoche dem klinischen Anwender zur Verfügung gestellt werden.

Typischerweise kommt im Rahmen der vorliegenden Erfindung ein mit dem Radionuklid markiertes Produkt zum Einsatz kommt, welches wenigstens eine Chelatorkomponente und wenigstens eine Targetmolekülkomponente enthält, wobei die Targetmolekülkomponente in der Lage ist, an ein spezifisches Target in oder an eine Zielzelle zu binden und wobei die Chelatorkomponente und die Targetmolekülkomponente zu einer Chelator-Targetmolekül-Einheit kovalent aneinander gebunden sind und das Radionuklid koordinativ an die Chelatorkomponente gebunden ist. Hierdurch ist für jedes Radionuklid und Target die jeweils optimale chemische Struktur gegeben.

Vorzugsweise wird ein Produkt eingesetzt, bei welchem als Chelatorkomponente ein cyclisches Polyazasystem mit 4 bis 8 N-Atomen eingesetzt wird. Derartige Chelatoren haben sich für eine Reihe von Übergangsmetallen als vorteilhaft erwiesen. Sie können bei der Synthese der komplexen Produkte auch problemlos reversibel mit Schutzgruppen versehen werden, um unerwünschte Nebenreaktionen zu vermeiden.

Eine bevorzugte pharmazeutisch akzeptable Chelatorkomponente ist die kommerziell verfügbare 1,4,7,10-Tetraazacyclododecan-1,4,7,10-tetraessigsäure [DOTA] oder eine ihrer ionischen Formen oder pharmazeutisch akzeptablen Salze.

Die im Rahmen der vorliegenden Erfindung eingesetzten Targetmolekülkomponenten richten sich im Grundsatz nach dem verfolgten medizinischen Anwendungszweck.

Für die Tumortherapie und -diagnostik haben sich beispielsweise Somatostatin-analoge Peptide als nützlich erwiesen. Besonders bevorzugt, sind Targetmolekülkomponenten, welche ausgewählt sind, aus der Gruppe, bestehend aus: Peptiden, insbesondere cyclischen Peptiden mit 4 bis 20 Aminosäuren, wobei wenigstens eine Aminosäure eine D-Aminosäure ist, insbesondere D-Phenylalanin; und einem Protein, insbesondere einem Rezeptorprotein, vorzugsweise PSMA.

Der Ersatz von L-Aminosäuren durch D-Aminosäure-Enatiomere in den Targetmolekülkomponenten hat den Grund, dass hierdurch die gekuppelten Target-Peptide weniger den in vivo Angriffen von Proteasen oder Peptidasen ausgesetzt sind, weil diese typischerweise als natürliche Substrate die physiologisch vorkommenden L-Aminosäure-Peptide und -Proteine abbauen. Durch die Integration von D-Aminosäuren in die Targetmolekülkomponente verlängert sich somit die biologische Halbwertszeit signifikant, weil der proteolytische Abbau hierdurch signifikant verzögert ist.

Als Somatostatin-analoge Verbindung hat sich ein Octreotid oder ein Octreotid-Analogon, insbesondere TOC, als Targetmolekül herausgestellt.

Ein besonders bevorzugtes System ist es, als Chelator-Targetmolekül-Einheit Edotreotid (DOTATOC) oder ein pharmazeutisch akzeptables Salz davon, zu verwenden. Insbesondere bevorzugt ist es im Rahmen der vorliegenden Erfindung als Radionuklid enthaltendes Produkt ein [n.c.a. Lu-177]Lu-DOTATOC einzusetzen. Dieses mit dem erfindungsgemäßen Verfahren herstellbare Produkt bindet selektiv an das Tumorgewebe von sogenannten GEP-NETs und zerstört es durch die Abgabe zytotoxischer, ionisierender Strahlendosen. Neuroendokrine Tumoren (NETs) des gastoenteropankreatischen Systems (GEP) [GEP-NETs] umfassen eine Gruppe von Tumoren mit großen Unterschieden in ihrem Wachstums- und hormonellen Verhaltens. Ebenso breit ist das Spektrum der klinischen Verläufe: Auf der einen Seite stehen gutartige Tumoren, die etwa als Zufallsbefunde der Bildgebung oder histologischen Aufarbeitung von OP-Präparaten diagnostiziert werden, auf der anderen Seite kommt es zu klinisch ungünstigen Verläufen durch schnell wachsende Tumoren.

Insbesondere für diese Tumorgattung kommt der vorliegenden Erfindung eine besondere klinische Bedeutung zu.

Das mit dem erfindungsgemäßen Verfahren zur Verfügung gestellte [n.c.a. Lu-177]Lu-DOTATOC wird derzeit von der Anmelderin der vorliegenden Erfindung in der klinischen Phase III (Stand Februar 2021) als Solucin^{®} zur Behandlung von GEP-Nets in der sogenannten COMPETE-Studie getestet. Das Präparat Solucin^{®} besteht aus zwei molekularen Komponenten: Zum einen aus dem Targeting-Molekül Edotreotid (DOTATOC), einem Somatostatin-Analogon, und zum anderen aus dem von der EMA zugelassenen Beta-Strahler EndolucinBeta^{®} (no-carrier-added Lutetium-177, eingetragene Marke der ITM Isotopen Technologien München AG).

Andere Produkt-Peptidkombinationen, welche erfindungsgemäß zum Einsatz kommen können, beinhalten beispielsweise [Lu-177]Lu-PSMA zur Therapie und Diagnose des Prostatakarzinoms.

Bevorzugt werden neben den aktiven pharmazeutischen Ingredienzien (API) Hilfsstoffe wie allgemeine Excipienten und/oder Puffersysteme in der Stammlösung und der Verdünnungslösung verwendet.

Vorteilhaft kann als Puffersystem ein Ascorbinsäure/Ascorbatpuffer verwendet werden, welches sich vielfach in der Praxis bewährt hat.

Zur Durchführung des erfindungsgemäßen Verfahrens wird vorteilhaft ein unter Unterdruck stehendes leckagefreies Fluidiksystem eingesetzt, weil hierdurch eine radioaktive Kontamination vollständig vermieden werden kann.

In bevorzugter Ausführungsform erfolgt die Umsetzung des Radionuklids zu dem markierten Produkt mittels Präkursoren, die mit dem das Radionuklid enthaltenden Konzentrat mittels eines in das Fluidiksystem eingeschleiften temperaturgeregelten Reaktors. Durch die Temperaturregelung können unterschiedliche für das jeweilige chemische System erforderliche Reaktionsbedingungen realisiert werden. So kann eine gewünschte Umsetzung beispielsweise produktspezifisch bei einer Temperatur von 20°C bis 100°C und während einer Zeit von 5 min bis zu mehreren Stunden in dem Reaktor erfolgen.

Zur Erfüllung der nach GMP üblichen Hygienestandards wird jede Teilabfüllung vor Einlauf in ein pharmazeutisch akzeptables Vial über einen Sterilfilter geführt. Hierzu setzt man vorteilhaft handelsübliche belüfteter Sterilfilter mit einem Porendurchmesser von 220 nm oder ein Mehrschichtfilter mit einem Porendurchmesser von 450 nm einer ersten Schicht und einem Porendurchmesser einer zweiten Schicht von 220 nm ein.

Typischerweise ist an dem verwendeten Sterilfilter auf dessen unsteriler Seite eine By-Pass-Leitung zurück in ein Bulkgefäß vorgesehen, wodurch einerseits die nächste Chargen-Abfüllung und/oder Teilabfüllung vorbereitet wird und andererseits in vorteilhafter Weise zwischen den einzelnen Chargen über die By-Pass-Leitung ein im Wesentlichen verlustfreies Spülen einer Abfüllleitung und des Sterilfilters auf dessen unsteriler Seite durchgeführt werden kann, so dass keine Radioaktivität unkontrolliert in die nächste Abfüllung verschleppt wird und die Kalibrierung auf den ART auch für die nachfolgende Abfüllung korrekt ist.

Zur Sicherung der Qualität werden GMP-konform von jeder Charge Proben für eine Qualitätskontrolle entnommen. Hierbei hat sich herausgestellt, dass die Chargen jeweils für sich genommen ein homogenes Bild zeigen.

Bevorzugt wird das erfindungsgemäße Verfahren eingesetzt, um [Lutetium-177]Lu-DOTATOC (SOLUCIN^{®}) als Radionuklid enthaltendes Produkt herzustellen, wobei ein Konzentrat eingesetzt wird, welches bezogen auf den Kalibrierzeitpunkt (ART) folgende Aktivität und Bestandteile enthält:

| | |
|---|---|
| - Lutetium-177 | 7.5 ± 0.7 GBq |
| - Edotreotid (DOTATOC) | 150 ± 15 µm |
| - Ascorbinsäure | 20 ± 2 mg |
| - Na-Ascorbat | 80 ± 8 mg |
| - Reinstwasser | 1.00 ± 0.01 ml |
| - 0.1 M Na-Ascorbat-Verdünnungslösung | 18.0 ± 2 ml. |

Weitere Vorteile und Merkmale der vorliegenden Erfindung ergeben sich aufgrund der Beschreibung von Ausführungsbeispielen sowie anhand der Zeichnung.

Es zeigt:
- Fig. 1: ein Schema zur Bereitstellung eines Radiopharmazeutikums zu jedem Anwendungszeitpunkt innerhalb einer Arbeitswoche durch mindestens 5 Arzneimittelchargen durch einen Hersteller. A) Herstellung täglich. B) Herstellung gepoolt. ART = Activity Reference Time (Kalibrierzeitpunkt);
- Fig. 2: ein Schema zur Bereitstellung eines Radiopharmazeutikums zu einem bestimmten Anwendungszeitpunkt innerhalb einer Arbeitswoche mittels einer großen Arzneimittelcharge durch einen Hersteller;
- Fig. 3: ein Abfüllschema gemäß Alternative 1;
- Fig. 4: ein Abfüllschema gemäß einem erfindungsgemäßen Verfahren (Alternative 2);
- Fig. 5: ein Schema zur Bereitstellung eines Radiopharmazeutikums zu mehreren Anwendungszeitpunkten innerhalb einer Arbeitswoche durch einen einzigen Syntheseansatz gemäß der Erfindung;
- Fig. 6: eine schematische Anordnung einer Syntheseapparatur und Aufbau der Fluidik einer Vorrichtung zur Durchführung des erfindungsgemäßen Verfahrens;
- Fig. 7: ein Flussdiagramm zur Herstellung von ART-spezifischen Abfüllungen nach Aufbau in Abbildung 6; und
- Fig. 8: ein Schema eines Spülprozesses einer Abfülllinie und eines Sterilfilters über einen By-Pass.

### Ausführungsbeispiel

Die vorliegende Erfindung wird ohne Einschränkung hierauf am Beispiel eines Verfahrens zur Herstellung von Solucin^{®} (eingetragene Marke der ITM Isotopen Technologien München AG) beschrieben. Wirkstoff des pharmazeutischen Präparates Solucin^{®} ist [n.c.a. Lu-177]Lu-DOTATOC.

Selbstverständlich lassen sich die Prinzipien der vorliegenden Erfindung auch auf andere radiomarkierten Arzneimittel wie bspw. [Lu-177]Lu-PSMA übertragen. Dasselbe gilt für die Verwendung mit anderen kurzlebigen Radionukliden.

In Fig. 1 ist zur Verdeutlichung die Bereitstellung eines Radiopharmazeutikums nach dem Stand der Technik zu jedem Anwendungszeitpunkt innerhalb einer Arbeitswoche durch mindestens 5 Arzneimittelchargen durch einen Hersteller schematisch gezeigt. Fig.1 A) zeigt die Situation bei täglicher Herstellung, während Fig. 1 B) die Situation bei gepoolter Herstellung wiedergibt. ART ist die "Activity Reference Time", also der Kalibrierzeitpunkt.

Fig. 2 zeigt die Herstellungssituation in schematischer Darstellung, wenn ein Radiopharmazeutikum nur zu einem bestimmten Anwendungszeitpunkt innerhalb einer Arbeitswoche - hier mittwochs - vom Hersteller bereitgestellt wird.

Im Gegensatz zu den Verfahrensführungen des Standes der Technik erlaubt es das erfindungsgemäße Verfahren durch einen einzigen Herstellprozess die gleichbleibende Zusammensetzung des gewünschten radionuklidmarkierten Arzneimittels zu allen Anwendungszeitpunkten innerhalb der Haltbarkeit zu gewährleisten (Tabelle 1). Das Abfüllschema gemäß der vorliegenden Erfindung ist in Fig. 4 gezeigt und die Bereitstellungssituation eines Radiopharmazeutikums zu mehreren Anwendungszeitpunkten innerhalb einer Arbeitswoche durch einen einzigen Syntheseansatz gemäß der vorliegenden Erfindung ist in Fig. 5 gezeigt.

**Tabelle 1: Beispielhafte Spezifikation von Solucin^{®} zum Kalibrierzeitpunkt (ART)**

| **Bestandteil** | **Menge pro Gefäß (Vial) / Wert am ART** |
|---|---|
| Lutetium (¹⁷⁷Lu) | 7.5 ± 0.7 GBq |
| Edotreotide | 150 ± 15 µg |
| Ascorbinsäure | 20 ± 2 mg |
| Natriumiumascorbat | 80 ± 8 mg |
| Hochreines Wasser | 1.00 ± 0.01 ml |
| Formulation, 0.1 M Natriumascorbat | 18 ± 2 ml |

Die besondere Konfiguration der Prozessfluidik und die Zusammensetzung der eingesetzten Reagenzien gewährleistet eine kompakte, leicht skalierbare und transferierbare Synthese. Dadurch gelingt es die Herstellung auf einen einzigen Bulkansatz zu reduzieren und die Vorteile einer täglichen Verfügbarkeit zu gewährleisten.

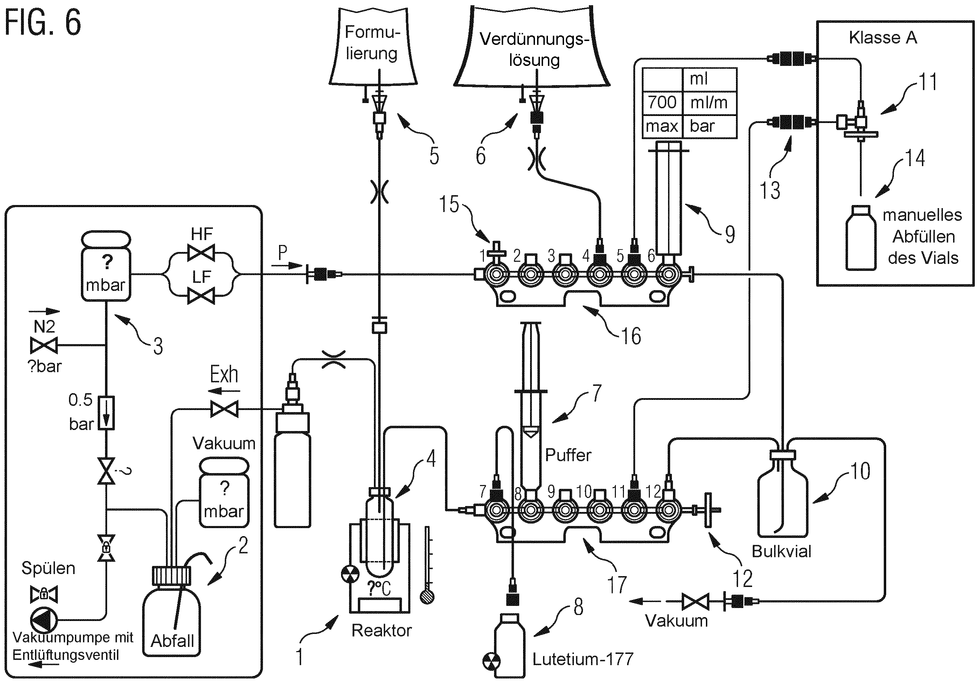

Fig. 6 zeigt den schematischen Aufbau der Fluidik, sowie die weiteren Vorrichtungen und Anordnungen zur Synthese, mit:
Einem regelbarem Temperaturelement 1 zum Heizen bis auf 100°C innerhalb von 5 min;
einer regelbaren Vakuumpumpe 2 bis zu 200 mbar mit Pneumatik und Entlüftungsventilen;
einer regelbaren Stickstoff-Pneumatik 3, welche einen Druck von bis zu 6 bar liefert;
einem Reaktor 4 aus Glas- oder Plastik mit 2-3 Anschlüssen;
einem Behälter 5 oder Beutel für die Formulierungslösung;
einem Behälter 6 oder Beutel für die Verdünnungslösung;
einem Reaktionspuffer in einer Spritze 7, Vial oder Behälter;
einer Vorlage 8 für einen radiochemischen Präkursor, im Beispielsfalle Lu-177;
einer Abfüllspritze 1-20 ml;
einem Bulkaufbewahrungs- und Mischgefäß 10;
einem belüfteter Sterilfilter 0.22 µm bzw. Mehrschichtfilter 0.45 µm, 0.22 µm;
einem Luftfilter 0.22 µm;
einer By-Pass Leitung 13 mit aseptischen Konnektor;
einer open- oder closed-Vial Abfüllstation 14;
einem Luftfilter 0.22 µm;
einer ersten Hahnenbank 16 mit 2-3 Wegeventilen; und
einer zweiten Hahnenbank 17 mit 2-3 Wegeventilen.

Der Transfer von Flüssigkeiten kann durch den Aufbau leckagesicher mittels Unterdruck erfolgen. Die Spritzenpumpe 9 dient lediglich zu Abfüllzwecken und zur Verdünnung des Bulkansatzes zu den entsprechenden ARTs. Die Herstellung der ART spezifischen Bulklösungen kann wie im Folgenden durchgeführt werden:
1. Herstellen des radiomarkierten Konzentrats durch Zugabe von Pufferlösung zur radiochemischen und chemischen Präkursor und Erhitzen in einem entsprechenden Reaktor. Temperatur und Zeit sind produktspezifisch und können variieren zwischen Raumtemperatur und 100°C sowie 5 Minuten bis mehrere Stunden.
2. Herstellung des spätesten ART (Bspw. ART+4Tage nach Herstellung) durch Zugabe der Formulierungslösung und mischen im Bulkgefäß 10 zum abfüllbereiten Arzneimittel. ART+4 bedeutet in diesem Fall, dass das Arzneimittel am Kalibrierzeitpunkt (ART) im Beispielsfalle die Spezifikation gemäß Tabelle 1 erfüllt.
3. Verlustfreies Spülen der Abfüllleitung und des Sterilfilters 11 (unsterile Seite) über By-Pass-Leitung 13 zurück in das Bulkgefäß 10 zur Vorbereitung der Abfüllung.
4. Abfüllen ART+4 Tage und/oder Probenzug zur Qualitätskontrolle.
5. Nach erfolgter Abfüllung kann optional ein weiterer Probenzug erfolgen bzw. auch ein Filterintegritätstest mit Hilfe der Spritzenpumpe 7 oder N2-Pneumatik 3 erfolgen.
6. Die Abfüllspritze 9 wird verwendet, um den Bulk Art+4 Tage auf den ART+3 Tage (bzw. ART+4-X Tage) mit Hilfe der Verdünnungslösung (fillup solution) zu verdünnen.
7. Anschließend erfolgen das Homogenisieren des Bulkansatzes und das Spülen der By-Pass-Leitung 13 analog zum in Punkt 3 beschriebenen Prozess.
8. Das Abfüllen des Bulkansatzes ART+3 Tage etc. erfolgt dann analog wie oben beschrieben.

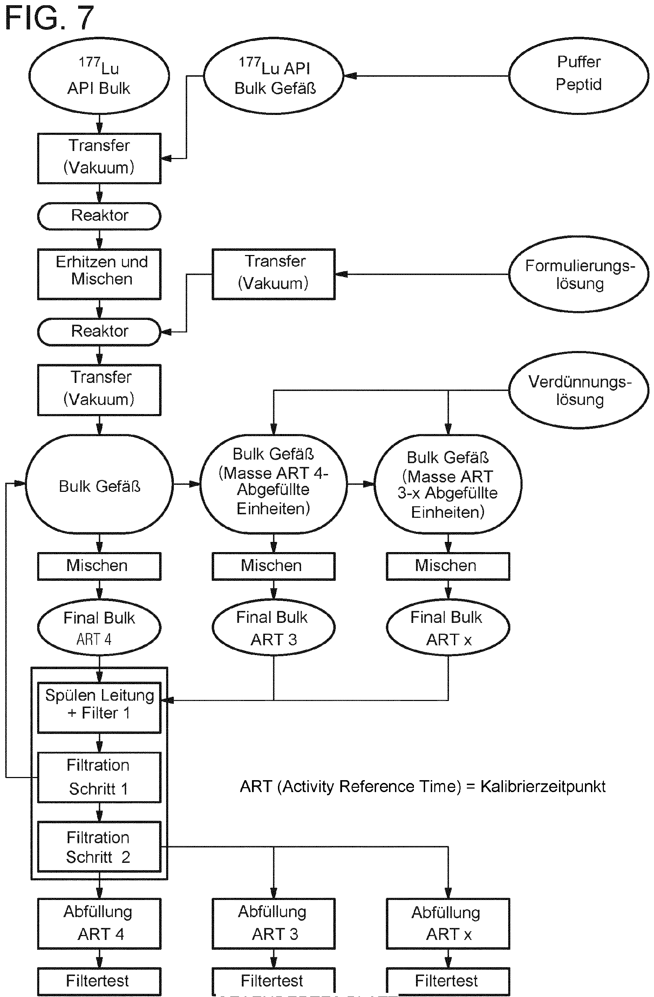

Das Flussdiagramm gemäß Fig. 7 gibt einen schematischen Überblick über den erfindungsgemäßen Herstellungsprozess von ART-spezifischen Abfüllungen mit einer Fluidik gemäß Fig. 6.

Die Zusammensetzung der Verdünnungslösung und die Zugabemengen zu den einzelnen ARTs lassen sich leicht an Hand der Spezifikation des Radiopharmazeutikums errechnen. Für das Beispiel Solucin^{®} ([n.c.a.¹⁷⁷Lu]Lu-DOTATOC) in Tabelle 1 ergeben sich die Daten gemäß Tabelle 2 und 3:

**Tabelle 2: Zusammensetzung des finalen Radiopharmazeutikums und der Verdünnungslösung.**

| **Gehalt** | **Spezifizierte Konzentration am ART** | **Konzentration der Verdünnungslösung** |
|---|---|---|
| Lutetium (¹⁷⁷Lu) | 0.64 GBq/mL ± 10 % | - |
| Edotreotide | max. 8.33 µg/mL | max. 8.33 µg/mL |
| Natriumascorbat | 0.1 M | 0.1 M |

**Tabelle 3: Anteil der Art+4 Tage Formulierung an den entsprechenden ART+4-X Tage Formulierungen.**

| ART | Aktivität am ART | Aktivität zum Abfüllzeitpunkt | Vol. des ART+4 | Verdünnungslösung aus Tabelle2 | Konzentration am ART | DOTATOC |
|---|---|---|---|---|---|---|
| Tage | [GBq] | [GBq] | [mL] | [mL] | [GBq/mL] | [µg] |
| 0 | 7.7 | 7.7 | 12.1 | 5.9 | 0.64 | 150 |
| 1 | 7.7 | 8.5 | 13.3 | 4.7 | 0.64 | 150 |
| 2 | 7.6 | 9.4 | 14.7 | 3.3 | 0.64 | 150 |
| 3 | 7.7 | 10.5 | 16.4 | 1.6 | 0.64 | 150 |
| 4 | 7.6 | 11.5 | 18.0 | 0.0 | 0.64 | 150 |

Durch eine entsprechende Auftragsplanung und durch eine einfach validierbare Kalkulationstabelle ist eine Produktionsplanung mit dem erfindungsgemäßen Verfahren leicht umsetzbar.

Maßgeblich entscheidend für die Umsetzung der vorliegenden Erfindung ist ein By-Pass über By-Pass-Leitung 13 vom Sterilfilter 11 zurück zum Bulkgefäß 10. Durch die Zirkulierung der Lösungen zwischen der Abfülllinie/Sterilfilter und dem Bulkgefäß lässt sich ein verlustfreies Abfüllen aller spezifischen ARTs in einer Anlage ökonomisch und abfallwirtschaftlich sinnvoll gestalten. Um die spezifischen ARTs in einem Ansatz darzustellen sind entweder zwei Abfülllinien notwendig wie in Alternative 1 oder aber man müsste die Abfüllleitung nach jedem ART entleeren und neu spülen, wobei bei sehr langen Leitungen (zu erwarten, da die Reinraumklassen aufgrund neuer Vorschriften von C zu A gewechselt werden) und somit hohe Verluste und zusätzlicher Radioaktivabfall auftreten würde. Aus diesem Grund weist insbesondere der By-Pass einen besonderen Vorteil für die technische Lösung der Aufgabenstellung auf.

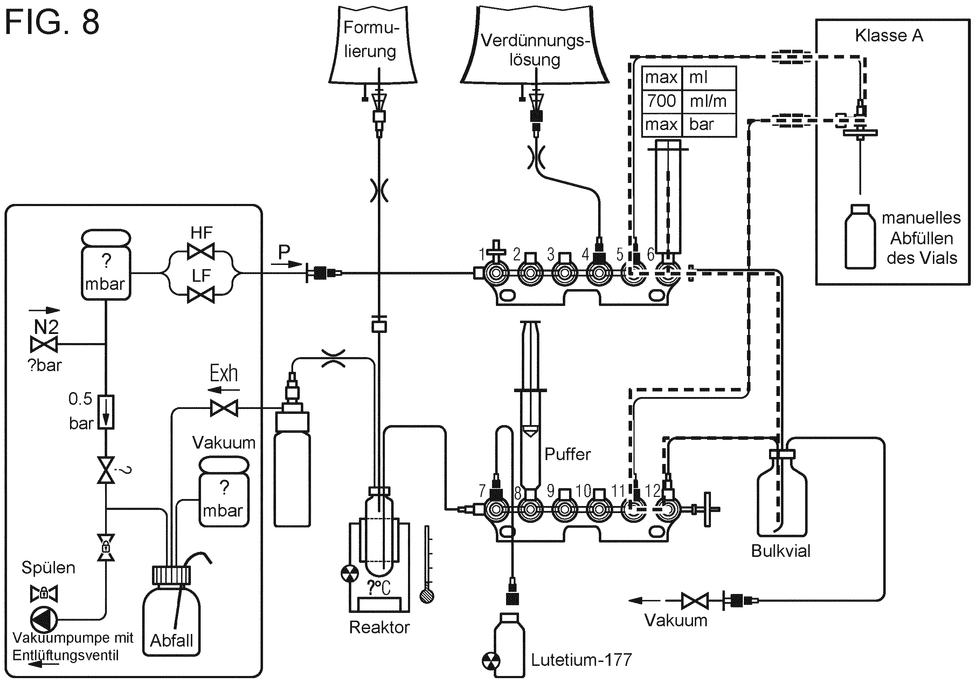

Fig. 8 zeigt die Zirkulation der Bulklösung und den Spülvorgang über die gestrichelt dargestellten Leitungswege. Durch diesen Spülvorgang wird eine verlustfreie Abfüllung und eine homogene Abfülllösung nach Einstellung der spezifischen ARTs garantiert. Bei dem vorliegenden beispielhaften Verfahren kann, insbesondere durch die By-Pass-Leitung 13 vor dem Sterilfilter 11 zurück in das Bulkgefäß 10, erreicht werden, dass auch die Leitungswege gespült und mit homogener Lösung gefüllt werden. Die By-Pass-Leitung 13 wird durch ein Ventil der ersten Hahnenbank 16 beim Spülen geöffnet und beim Abfüllen geschlossen. Der natürliche Widerstand des Sterilfilters 11 verhindert bei geöffneter By-Pass-Leitung 13, dass Flüssigkeit über Sterilfilter 11 austritt und lenkt die Fließrichtung des Mediums in das Bulkgefäß 10.

## Patentansprüche

1. Verfahren zur Herstellung von Radionuklide enthaltenden Produkten mit einer gleichen gewünschten Aktivität der Radioaktivität zu unterschiedlichen Anwendungszeitpunkten (ART+1, ART+2, ART+3, ART+4), bezogen auf einen gegebenen Kalibrierzeitpunkt (ART),
**dadurch gekennzeichnet, dass**
- ein Radionuklid enthaltendes Konzentrat zu einem gewünschten mit dem Radionuklid markierten Produkt umgesetzt wird und so eine Stammlösung erhalten wird, welche neben dem radionuklidmarkierten Produkt sämtliche für den Anwendungszweck benötigten weiteren Bestandteile enthält, wobei
- das gewünschte Radionuklid in der Stammlösung in einer solchen Aktivität enthalten ist, dass eine Mehrzahl von gewünschten Chargen mit jeweils einer definierten Anzahl von Teilabfüllungen zu einem Abfüllzeitpunkt aus der Stammlösung erhältlich ist, wobei jede Charge von Teilabfüllungen zu unterschiedlichen Anwendungszeitpunkten (ART+1, ART+2, ART+3, ART+4) jeweils eine gleiche Aktivität des Radionuklids, bezogen auf den Kalibrierzeitpunkt (ART), aufweist;
- die Aktivität des radionuklidmarkierten Produktes in der Stammlösung auf einen spätesten gewünschten Anwendungszeitpunkt (ART+4) eingestellt wird;
- aus der das radionuklidmarkierte Produkt enthaltenden Stammlösung eine erste Charge von Teilabfüllungen zu einem ersten vor dem Anwendungszeitpunkt liegenden Abfüllzeitpunkt entnommen wird, welche eine auf den spätesten Anwendungszeitpunkt (ART+4) eingestellte Aktivität aufweist, welche zu ihrem tatsächlichen Anwendungszeitpunkt der Aktivität zum Kalibrierzeitpunkt (ART) entspricht;
- eine Verdünnungslösung bereitgestellt wird, welche mit Ausnahme des radionuklidmarkierten Produktes sämtliche für den Anwendungszweck benötigten weiteren Bestandteile enthält;
- die verbleibende, auf den spätesten gewünschten Anwendungszeitpunkt (ART+4) eingestellte Stammlösung mit der Verdünnungslösung derart verdünnt wird, dass zum Abfüllzeitpunkt eine gewünschte verminderte Aktivität, bezogen auf den spätesten Anwendungszeitpunkt (ART+4) eingestellt wird, so dass für den Einsatz zum vorhergehenden Anwendungszeitpunkt eine zweite Charge von Teilabfüllungen entnommen wird, welche eine auf einen früheren Anwendungszeitpunkt (ART+3) eingestellte Aktivität aufweist, welche zu ihrem tatsächlichen Anwendungszeitpunkt der Aktivität zum Kalibrierzeitpunkt (ART) entspricht;
- die verbleibende, auf den früheren Anwendungszeitpunkt (ART+3) eingestellte Stammlösung mit der Verdünnungslösung schrittweise solange weiter verdünnt wird, bis der Anwendungszeitpunkt dem Kalibrierzeitpunkt (ART) entspricht; und
- jeweils weitere Chargen von Teilabfüllungen zu jedem weiteren Anwendungszeitpunkt (ART+2, ART+1) entnommen werden, welche eine auf den jeweiligen Anwendungszeitpunkt (ART+2, ART+1) eingestellte Aktivität aufweisen, wobei die letzte Charge die Aktivität des Kalibrierzeitpunktes (ART) aufweist.

2. Verfahren nach Anspruch 1, **dadurch gekennzeichnet, dass** das Radionuklid ausgewählt wird, aus der Gruppe, bestehend aus: Gallium-68, Yttrium-90, Molybdän-99, Indium-111, Gadolinium-146, Gadolinium 147, Holmium-166, Lutetium-177, Wolfram-188, Rhenium-188, Bismut-205, Bismut-206 und Thorium-227.

3. Verfahren nach Anspruch 1 oder 2, **dadurch gekennzeichnet, dass** ein mit dem Radionuklid markiertes Produkt zum Einsatz kommt, welches wenigstens eine Chelatorkomponente und wenigstens eine Targetmolekülkomponente enthält, wobei die Targetmolekülkomponente in der Lage ist, an ein spezifisches Target in oder an einer Zielzelle zu binden und wobei die Chelatorkomponente und die Targetmolekülkomponente zu einer Chelator-Targetmolekül-Einheit kovalent aneinander gebunden sind und das Radionuklid koordinativ an die Chelatorkomponente gebunden ist.

4. Verfahren nach Anspruch 3, **dadurch gekennzeichnet, dass** ein Produkt eingesetzt wird, bei welchem als Chelatorkomponente ein cyclisches Polyazasystem mit 4 bis 8 N-Atomen eingesetzt wird.

5. Verfahren nach Anspruch 4, **dadurch gekennzeichnet, dass** als Chelatorkomponente 1,4,7,10-Tetraazacyclododecan-1,4,7,10-tetraessigsäure [DOTA] oder eine seiner ionischen Formen eingesetzt wird.

6. Verfahren nach einem der vorhergehenden Ansprüche 1 bis 5, **dadurch gekennzeichnet, dass** die Targetmolekülkomponente ausgewählt wird, aus der Gruppe, bestehend aus: Peptiden, insbesondere cyclischen Peptiden mit 4 bis 20 Aminosäuren, wobei wenigstens eine Aminosäure eine D-Aminosäure ist, insbesondere D-Phenylalanin; und einem Protein, insbesondere einem Rezeptorprotein, vorzugsweise PSMA.

7. Verfahren nach Anspruch 6, **dadurch gekennzeichnet, dass** als Targetmolekül eine Somatostatin-analoge Verbindung, insbesondere ein Octreotid oder ein Octreotid-Analogon, insbesondere TOC verwendet wird.

8. Verfahren nach einem der vorhergehenden Ansprüche 1 bis 7, **dadurch gekennzeichnet, dass** als Chelator-Targetmolekül-Einheit Edotreotid (DOTATOC) oder ein pharmazeutisch akzeptables Salz davon, verwendet wird.

9. Verfahren nach einem der vorhergehenden Ansprüche 1 bis 8, **dadurch gekennzeichnet, dass** als Radionuklid enthaltendes Produkt ein [Lu-177]Lu-DOTATOC oder ein [Lu-177]Lu-PSMA eingesetzt wird.

10. Verfahren nach einem der vorhergehenden Ansprüche 1 bis 9, **dadurch gekennzeichnet, dass** neben den aktiven pharmazeutischen Ingredienzien (API) Hilfsstoffe wie allgemeine Excipienten und/oder Puffersysteme in der Stammlösung und der Verdünnungslösung verwendet werden.

11. Verfahren nach Anspruch 10, **dadurch gekennzeichnet, dass** als Puffersystem ein Ascorbinsäure/Ascorbatpuffer verwendet wird.

12. Verfahren nach einem der vorhergehenden Ansprüche 1 bis 11, **dadurch gekennzeichnet, dass** zur Durchführung des Verfahrens ein unter Unterdruck stehendes leckagefreies Fluidiksystem eingesetzt wird.

13. Verfahren nach Anspruch 12, **dadurch gekennzeichnet, dass** zur Umsetzung des Radionuklids zu dem markierten Produkt Präkursoren verwendet werden, die mit dem das Radionuklid enthaltenden Konzentrat mittels eines in das Fluidiksystem eingeschleiften temperaturgeregelten Reaktors (4) umgesetzt werden.

14. Verfahren nach Anspruch 13, **dadurch gekennzeichnet, dass** die Umsetzung im Reaktor (4) produktspezifisch bei einer Temperatur von 20°C bis 100°C und während einer Zeit von 5 min bis zu mehreren Stunden erfolgt.

15. Verfahren nach einem der vorhergehenden Ansprüche 1 bis 14, **dadurch gekennzeichnet, dass** jede Teilabfüllung vor Einlauf in ein pharmazeutisch akzeptables Vial (14) über einen Sterilfilter (11) geführt wird.

16. Verfahren nach Anspruch 15, **dadurch gekennzeichnet, dass** als Sterilfilter (11) ein belüfteter Sterilfilter mit einem Porendurchmesser von 220 nm oder ein Mehrschichtfilter im einem Porendurchmesser von 450 nm einer ersten Schicht und einem Porendurchmesser einer zweiten Schicht von 220 nm verwendet wird.

17. Verfahren nach Anspruch 16, **dadurch gekennzeichnet, dass** an dem Sterilfilter (11) auf dessen unsteriler Seite eine By-Pass-Leitung (13) zurück in ein Bulkgefäß (10) zur Vorbereitung der nächsten Chargen-Abfüllung und/oder Teilabfüllung eingesetzt wird.

18. Verfahren nach einem der vorhergehenden Ansprüche 1 bis 17, **dadurch gekennzeichnet, dass** von jeder Charge Proben für eine Qualitätskontrolle entnommen werden.

19. Vorrichtung zur Durchführung des Verfahrens gemäß wenigstens einem der Ansprüche 1 bis 18 mit einem Fluidiksystem, an welches die folgenden Bauteile angeschlossen sind:
wenigstens ein Reaktor (4);
ein einstellbares Heizelement (1), das zum Heizen des Reaktors (4) dient;
eine einstellbare Vakuumpumpe (2) mit Pneumatik und Entlüftungsventilen;
eine einstellbare Inertgaspneumatik (3);
ein Behälter (5) für eine Formulierungslösung, welcher fluidisch mit dem Reaktor (4) verbunden ist;
ein Behälter (6) für eine Verdünnungslösung;
einem Reaktionspufferbehälter (7);
einem Vorlagebehälter (8) für einen radiochemischen Präkursor;
eine Abfülldosiereinrichtung (9);
ein Bulkaufbewahrungs- und Mischgefäß (10);
ein belüfteter Sterilfilter (11);
Luftfilter (12, 15);
einer By-Pass-Leitung (13) zwischen unsteriler Seite des Sterilfilters (11) und
dem über einen Dreiwegehahn damit fluidisch verbundenen Bulkaufbewahrungs- und Mischgefäß (10);
einer Abfülleinrichtung (14); sowie
einer ersten Hahnenbank (16) mit Mehrwegeventilen; und
einer zweiten Hahnenbank (17) mit Mehrwegeventilen;
wobei die erste Hahnenbank (16) in fluidischer Verbindung mit dem Luftfilter (15), dem Bulkaufbewahrungs- und Mischgefäß (10), der Inertgaspneumatik (3), dem Behälter (6), dem Sterilfilter (11) sowie der Abfülldosiereinrichtung (9) steht; und
wobei die zweite Hahnenbank (17) in fluidischer Verbindung mit dem Reaktor (4), dem Vorlagenbehälter (8), dem Reaktionspufferbehälter (7), der By-Pass-Leitung (13), dem Bulkaufbewahrungs- und Mischgefäß (10) und dem Luftfilter (12) steht wobei das Bulkaufbewahrungs- und Mischgefäß (10) in fluidischer Verbindung mit der Vakuumpumpe (2) steht.

## Claims

1. A method for the manufacture of radionuclide-containing products having an identical desired activity of radioactivity at different times of application (ART+1, ART+2, ART+3, ART+4), with respect to a given calibration time (ART), **characterized in that**
- a radionuclide-containing concentrate is converted to a desired radionuclide-labeled product and thus a bulk solution is obtained which, in addition to the radionuclide-labeled product, contains all further components required for the intended use, wherein
- the desired radionuclide is contained in the bulk solution in such an activity that a plurality of desired batches, each with a defined number of partial fillings, can be obtained from the bulk solution at a filling time, each batch of partial fillings at different times of application (ART+1, ART+2, ART+3, ART+4) in each case having an identical activity of the radionuclide, with respect to the calibration time (ART);
- the activity of the radionuclide-labeled product in the bulk solution is set to a latest desired time of application (ART+4);
- from the bulk solution containing the radionuclide-labeled product, a first batch of partial fillings is taken at a first filling time prior to the time of application, which has an activity set to the latest time of application (ART+4) which, at its actual time of application, corresponds to the activity at the calibration time (ART);
- a diluting solution is provided, which, with the exception of the radionuclide-labeled product, includes all other components required for the intended use;
- the remaining bulk solution set to the latest desired time of application (ART+4) is diluted with the diluting solution in such a way that, at the time of filling, a desired reduced activity based on the latest time of application (ART+4) is set, so that for use at the preceding time of application, a second batch of partial fillings is taken, which has an activity set to an earlier time of application (ART+3), which at its actual time of application, corresponds to the activity at the calibration time (ART);
- the remaining bulk solution set to the earlier time of application (ART+3) is continued to be diluted stepwise with the diluting solution until the time of application corresponds to the calibration time (ART); and
- further batches of partial fillings are respectively taken at each further time of application (ART+2, ART+1), which have an activity set to the respective time of application (ART+2, ART+1), the last batch having the activity of the calibration time (ART).

2. The method according to claim 1, **characterized in that** the radionuclide is selected from the group consisting of: gallium-68, yttrium-90, molybdenum-99, indium-111, gadolinium-146, gadolinium 147, holmium-166, lutetium-177, tungsten-188, rhenium-188, bismuth-205, bismuth-206 and thorium-227.

3. The method according to claim 1 or 2, **characterized in that** a radionuclide-labeled product is used, which contains at least one chelator component and at least one target molecule component, the target molecule component being capable of binding to a specific target in or on a target cell, and the chelator component and the target molecule component being bonded to each other covalently to form a chelator-target molecule unit, and the radionuclide being coordinately bound to the chelator component.

4. The method according to claim 3, **characterized in that** a product is used in which a cyclic polyaza system with 4 to 8 N atoms is used as a chelator component.

5. The method according to claim 4, **characterized in that** as the chelator component, 1,4,7,10-tetraazacyclododecane-1,4,7,10-tetraacetic acid [DOTA] or one of its ionic forms is used.

6. The method according to any of the preceding claims 1 to 5, **characterized in that** the target molecule component is selected from the group consisting of: peptides, in particular cyclic peptides with 4 to 20 amino acids, at least one amino acid being a D-amino acid, in particular D-phenylalanine; and a protein, in particular a receptor protein, preferably PSMA.

7. The method according to claim 6, **characterized in that** as the target molecule, a somatostatin analog compound, in particular an octreotide or an octreotide analog, in particular TOC, is used.

8. The method according to any of the preceding claims 1 to 7, **characterized in that** as chelator target molecule unit, edotreotide (DOTATOC) or a pharmaceutically acceptable salt thereof, is used.

9. The method according to any of the preceding claims 1 to 8, **characterized in that** as the radionuclide-containing product, an [Lu-177]Lu-DOTATOC or an [Lu-177]Lu-PSMA is used.

10. The method according to any of the preceding claims 1 to 9, **characterized in that** in addition to the active pharmaceutical ingredients (API), excipients such as general excipients and/or buffer systems are used in the bulk solution and the diluting solution.

11. The method according to claim 10, **characterized in that** as a buffer system, an ascorbic acid/ascorbate buffer is used.

12. The method according to any of the preceding claims 1 to 11, **characterized in that** for carrying out the method, a leakage-free fluidic system under negative pressure is used.

13. The method according to claim 12, **characterized in that** for conversion of the radionuclide to the labeled product, precursors are used, which are converted using the concentrate containing the radionuclide by means of a temperature-controlled reactor (4) introduced into the fluidic system.

14. The method according to claim 13, **characterized in that** the conversion is carried out in the reactor (4) in a product-specific manner at a temperature of 20°C to 100°C and for a time ranging from 5 min to several hours.

15. The method according to any of the preceding claims 1 to 14, **characterized in that** each partial filling is guided through a sterile filter (11) before entering a pharmaceutically acceptable vial (14).

16. The method according to claim 15, **characterized in that** as sterile filter (11), a ventilated sterile filter with a pore diameter of 220 nm or a multilayer filter having a pore diameter of 450 nm of a first layer and a pore diameter of a second layer of 220 nm is used.

17. The method according to claim 16, **characterized in that** on the sterile filter (11), on the non-sterile side thereof, a by-pass line (13) back into a bulk vessel (10) is used in preparation for the next batch filling and/or partial filling.

18. The method according to any of the preceding claims 1 to 17, **characterized in that** samples are taken from each batch for quality control.

19. An apparatus for carrying out the method according to at least one of claims 1 to 18 comprising a fluidic system to which the following components are connected:
at least one reactor (4)
an adjustable heating element (1) serving for heating the reactor (4);
an adjustable vacuum pump (2) with pneumatics and bleed valves;
adjustable inert gas pneumatics (3);
a vessel (5) for a formulation solution, which is connected to the reactor (4) in a fluidic manner;
a vessel (6) for a diluting solution;
a reaction buffer vessel (7);
a receiver vessel (8) for a radiochemical precursor;
a filling dosing device (9);
a bulk storage and mixing vessel (10);
a ventilated sterile filter (11);
an air filter (12, 15);
a by-pass line (13) between the non-sterile side of the sterile filter (11) and the bulk storage and mixing vessel (10) connected thereto in a fluidic manner via a three-way valve;
a filling device (14); as well as
a first cock bank (16) having multi-port valves; and
a second cock bank (17) having multi-port valves;
wherein the first cock bank (16) is in fluidic communication with the air filter (15),
the bulk storage and mixing vessel (10), the inert gas pneumatics (3), the vessel (6), the sterile filter (11) as well as the filling dosing device (9); and
wherein the second cock bank (17) is in fluidic communication with the reactor (4),
the receiver vessel (8), the reaction buffer vessel (7), the by-pass line (13), the bulk storage and mixing vessel (10) and the air filter (12), the bulk storage and mixing vessel (10) being in fluidic communication with the vacuum pump (2).

## Revendications

1. Procédé de préparation de produits contenant des radionucléides avec une même activité souhaitée de la radioactivité à différents moments d'application (ART+1, ART+2, ART+3, ART+4), par rapport à un moment d'étalonnage donné (ART),
**caractérisé en ce que**
- un concentré contenant un radionucléide est transformé en un produit souhaité marqué avec le radionucléide et une solution mère est ainsi obtenue, laquelle contient, outre le produit marqué avec le radionucléide, tous les composants supplémentaires nécessaires pour l'application prévue, dans lequel
- le radionucléide souhaité est contenu dans la solution mère dans une activité telle qu'une pluralité de lots souhaités avec respectivement un nombre défini de remplissages partiels à un moment de remplissage peut être obtenue à partir de la solution mère, dans lequel chaque lot de remplissages partiels à différents moments d'application (ART+1, ART+2, ART+3, ART+4) présente respectivement une même activité du radionucléide, par rapport au moment d'étalonnage (ART) ;
- l'activité du produit marqué avec le radionucléide dans la solution mère est réglée à un moment d'application souhaité le plus tardif (ART+4) ;
- un premier lot de remplissages partiels est prélevé de la solution mère contenant le produit marqué avec le radionucléide à un premier moment de remplissage antérieur au moment d'application, lequel présente une activité réglée au moment d'application le plus tardif (ART+4), lequel, à son moment d'application réel, correspond à l'activité au moment d'étalonnage (ART) ;
- une solution de dilution, laquelle contient tous les autres constituants nécessaires à l'application prévue à l'exception du produit marqué avec le radionucléide, est fournie ;
- la solution mère restante, réglée au moment d'application souhaité le plus tardif (ART+4), est diluée avec la solution de dilution de sorte qu'au moment de remplissage, une activité réduite souhaitée est réglée par rapport au moment d'application le plus tardif (ART+4), de sorte que pour l'utilisation au moment d'application précédent, un second lot de remplissages partiels est prélevé, lequel présente une activité réglée à un moment d'application antérieur (ART+3), lequel, à son moment d'application réel, correspond à l'activité au moment d'étalonnage (ART) ;
- la solution mère restante, réglée au moment d'application antérieur (ART+3), est diluée progressivement avec la solution de dilution jusqu'à ce que le moment d'application corresponde au moment d'étalonnage (ART) ; et
- respectivement des lots de remplissages partiels supplémentaires sont prélevés à chaque moment d'application supplémentaire (ART+2, ART+1), lesquels présentent une activité réglée au moment d'application respectif (ART+2, ART+1), dans lequel le dernier lot présente l'activité du moment d'étalonnage (ART).

2. Procédé selon la revendication 1, **caractérisé en ce que** le radionucléide est sélectionné à partir du groupe constitué par : Gallium-68, Yttrium-90, Molybdène-99, Indium-111, Gadolinium-146, Gadolinium 147, Holmium-166, Lutétium-177, Tungstène-188, Rhénium-188, Bismuth-205, Bismuth-206 et Thorium-227.

3. Procédé selon la revendication 1 ou 2, **caractérisé en ce qu'**un produit marqué avec le radionucléide, qui contient au moins un composant chélateur et au moins un composant molécule cible, est utilisé, dans lequel le composant molécule cible est capable de se lier à une cible spécifique dans ou sur une cellule cible et dans lequel le composant chélateur et le composant molécule cible sont liés de manière covalente l'un à l'autre pour former une unité chélateur-molécule cible et le radionucléide est lié par coordination au composant chélateur.

4. Procédé selon la revendication 3, **caractérisé en ce qu'**un produit est utilisé, dans lequel un système polyaza cyclique avec 4 à 8 atomes d'azote est utilisé en tant que composant chélateur.

5. Procédé selon la revendication 4, **caractérisé en ce qu'**un acide 1,4,7,10-tétraazacyclododécane-1,4,7,10-tétraacétique [DOTA] ou une de ses formes ioniques est utilisé en tant que composant chélateur.

6. Procédé selon l'une quelconque des revendications précédentes 1 à 5, **caractérisé en ce que** le composant molécule cible est sélectionné à partir du groupe constitué par :
des peptides, en particulier des peptides cycliques avec 4 à 20 acides aminés, dans lequel au moins un acide aminé est un D-aminoacide, en particulier la D-phénylalanine ; et une protéine, en particulier une protéine réceptrice, de préférence la PSMA.

7. Procédé selon la revendication 6, **caractérisé en ce qu'**un composé analogue à la somatostatine, en particulier un octréotide ou un analogue de l'octréotide, en particulier le TOC, est utilisé en tant que molécule cible.

8. Procédé selon l'une quelconque des revendications 1 à 7 précédentes, **caractérisé en ce que** l'édotréotide (DOTATOC) ou un de ses sels pharmaceutiquement acceptables est utilisé en tant qu'unité chélateur-molécule cible.

9. Procédé selon l'une quelconque des revendications précédentes 1 à 8, **caractérisé en ce qu'**un [Lu-177]Lu-DOTATOC ou un [Lu-177]Lu-PSMA est utilisé en tant que produit contenant un radionucléide.

10. Procédé selon l'une quelconque des revendications 1 à 9 précédentes, **caractérisé en ce que**, outre les ingrédients pharmaceutiques actifs (API), des adjuvants tels que des excipients généraux et/ou des systèmes tampons sont utilisés dans la solution mère et la solution de dilution.

11. Procédé selon la revendication 10, **caractérisé en ce qu'**un tampon acide ascorbique/ascorbate est utilisé en tant que système tampon.

12. Procédé selon l'une quelconque des revendications 1 à 11 précédentes, **caractérisé en ce qu'**un système fluidique sans fuite sous pression est utilisé pour la mise en œuvre du procédé.

13. Procédé selon la revendication 12, **caractérisé en ce que**, des précurseurs sont utilisés pour la conversion du radionucléide en produit marqué, qui sont convertis avec le concentré contenant le radionucléide au moyen d'un réacteur à température régulée (4) inséré dans le système fluidique.

14. Procédé selon la revendication 13, **caractérisé en ce que** la conversion dans le réacteur (4) s'effectue de manière spécifique au produit à une température de 20 °C à 100 °C et pendant une durée de 5 min à plusieurs heures.

15. Procédé selon l'une quelconque des revendications précédentes 1 à 14, **caractérisé en ce que** chaque remplissage partiel est guidé par l'intermédiaire d'un filtre stérile (11) avant l'entrée dans un flacon pharmaceutiquement acceptable (14).

16. Procédé selon la revendication 15, **caractérisé en ce qu'**un filtre stérile aéré est utilisé en tant que filtre stérile (11) avec un diamètre de pores de 220 nm ou un filtre multicouche avec un diamètre de pores de 450 nm d'une première couche et un diamètre de pores d'une seconde couche de 220 nm.

17. Procédé selon la revendication 16, **caractérisé en ce qu'**une conduite de dérivation (13) est utilisée sur le filtre stérile (11) sur son côté non stérile de retour dans un récipient en vrac (10) pour la préparation du remplissage de lot et/ou du remplissage partiel suivant.

18. Procédé selon l'une quelconque des revendications précédentes 1 à 17, **caractérisé en ce que** des échantillons sont prélevés sur chaque lot pour un contrôle de qualité

19. Dispositif pour la mise en œuvre du procédé selon au moins une quelconque des revendications 1 à 18, avec un système fluidique auquel les pièces suivantes sont raccordées :
au moins un réacteur (4) ;
un élément chauffant réglable (1), qui sert au chauffage du réacteur (4) ;
une pompe à vide réglable (2) avec système pneumatique et vannes de purge ;
un système pneumatique à gaz inerte réglable (3) ;
un conteneur (5) pour une solution de formulation, lequel est connecté fluidiquement au réacteur (4) ;
un conteneur (6) pour une solution de dilution ;
un conteneur tampon de réaction (7) ;
un conteneur de stockage (8) pour un précurseur radiochimique ;
un appareil de dosage de remplissage (9) ;
un récipient de stockage et de mélange en vrac (10) ;
un filtre stérile ventilé (11) ;
un filtre à air (12, 15) ;
une conduite de dérivation (13) entre le côté non stérile du filtre stérile (11) et le récipient de stockage et de mélange en vrac (10) connecté à celui-ci par un robinet à trois voies ;
un appareil de remplissage (14) ; ainsi que
un premier banc de robinets (16) avec des vannes à voies multiples ; et
un second banc de robinets (17) avec des vannes à voies multiples ;
dans lequel le premier banc de robinets (16) est en communication fluidique avec le filtre à air (15), le récipient de stockage et de mélange en vrac (10), le système pneumatique à gaz inerte (3), le conteneur (6), le filtre stérile (11) ainsi que l'appareil de dosage de remplissage (9) ; et
dans lequel le second banc de robinets (17) est en communication fluidique avec le réacteur (4), le conteneur de stockage (8), le conteneur tampon de réaction (7), la conduite de dérivation (13), le récipient de stockage et de mélange en vrac (10) et le filtre à air (12), dans lequel le récipient de stockage et de mélange en vrac (10) est en communication fluidique avec la pompe à vide (2).
